# EUROPÄISCHE PATENTANMELDUNG

(11) **EP 1 181 953 A2**
(43) Veröffentlichungstag der Anmeldung: **27.02.2002**
(21) Anmeldenummer: 01118061.9
(22) Anmeldetag: 25.07.2001
(51) Int. Cl.: A61N 1/372

(54) **Implantierbares hermetisch dichtes Gehäuse für eine implantierbare medizinische Vorrichtung sowie Herstellungsverfahren dafür**

(30) Priorität: 25.08.2000 DE 10041727
(71) Anmelder: Cochlear Limited, Lane Cove, NSW 2066 (AU)
(72) Erfinder: Leysieffer, Hans, Dr.-Ing., 82024 Taufkirchen (DE)
(74) Vertreter: Schwan - Schwan - Schorer

(57) **Zusammenfassung**

Die Erfindung betrifft ein implantierbares, hermetisch dichtes Gehäuse für Komponenten einer implantierbaren medizinischen Vorrichtung, wobei das Gehäuse eine hermetisch dichte Trennwand aufweist, welche das Gehäuse in eine erste Kammer, die einen Speicher für elektrische Energie für die Stromversorgung der medizinischen Vorrichtung aufnimmt, und eine zweite Kammer, die eine Elektronikeinheit aufnimmt, unterteilt. Ferner betrifft die Erfindung Herstellungsverfahren für ein solches Gehäuse.

## Beschreibung

Die vorliegende Erfindung betrifft ein implantierbares, hermetisch dichtes Gehäuse für Komponenten einer implantierbaren medizinischen Vorrichtung, welches einen Energiespeicher für die Stromversorgung der medizinischen Vorrichtung sowie eine Elektronik-einheit aufnimmt.

Aktive Implantate der vorliegenden Erfindung können insbesondere Systeme zur Rehabilitation einer Hörstörung sein, wie sie im folgenden Stand der Technik näher beschrieben sind.

Die Rehabilitation sensorischer Hörstörungen mit teilimplantierbaren, elektronischen Systemen hat in den letzten Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea Implantaten wird ein Reizelektroden-Array in die Cochlea eingeführt, das von einem elektronischen System angesteuert wird, wobei dieses hermetisch dichte und biokompatibel eingekapselte Elektronikmodul operativ im knöchernen Bereich hinter dem Ohr (Mastoid) eingebettet ist. Das elektronische System enthält jedoch im wesentlichen nur Dekodier- und Treiberschaltungen für die Reizelektroden; die akustische Schallaufnahme, die Wandlung dieses Schallsignals in elektrische Signale und deren weitere Verarbeitung erfolgt grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive

Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO) und ist mit einem Kabel mit dem Sprachprozessor verbunden. Solche Cochlea-Implantat-Systeme, deren Komponenten und Prinzipien der transkutanen Signalübertragung sind beispielhaft in folgenden Patentschriften beschrieben: US 5 070 535, US 4 441 210, EP 0 200 321, US 5 626 629. Verfahren zur Sprachaufbereitung und-Kodierung bei Cochleaimplantaten sind beispielsweise in folgenden Patentschriften angegeben: EP 0 823 188, EP 0 190 836, US 5 597 380, US 5 271 397, US 5 271 397, US 5 095 904, US 5 601 617, US 5 603 726.

Neben der Rehabilitation gehörloser beziehungsweise ertaubter Patienten mit Cochlea Implantaten existieren seit geraumer Zeit Ansätze, Patienten mit einer sensorineuralen Hörstörung, die operativ nicht behebbar ist, mit teil- beziehungsweise vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen beziehungsweise hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie z.B. durch elektromagnetische und piezoelektrische Systeme. Der Vorteil dieser Verfahren wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft in Yanigahara und Suzuki et al. (Arch Otolaryngol Head Neck, Surg-Vol 113, 1987, S. 869-872; Hoke, M. (ed), Advances in Audiology, Vol. 4, Karger Basel, 1988), Zeitschrift HNO 46:27-37 (1998), Lehner et al. "Kaltfließende Elemente zur Ankopplung eines implantierbaren Hörgerätewandlers an Gehörknöchelchen oder Perilymphe", HNO 46:121-128 (1998), Baumann et al. "Grundlagen der Energieversorgung vollständig implantierbarer Hörgeräte für Innenohrschwerhörige", HNO 46:311-323 (1998), Lehner et al. "Ein osseointegrierter Mikromanipulator als Halterung für implantierbare Hörgerätewandler" HNO 46:507-512 (1998), Lehner et al. "Ein Mikromanipulator für intraoperative vibratorische Hörprüfungen mit einem implantierbaren Hörgerätewandler", HNO 46:844-852 (1998), Zenner et al. "Erste Implantation eines vollständig implantierbaren elektronischen Hörsystems bei Patienten mit Innenohrschwerhörigkeit", HNO 46:853-863 (1998), Leysieffer et al. "Ein vollständig implantierbares Hörsystem für Innenohrschwerhörige: TICA LZ 3001" und in zahlreichen Patentschriften beschrieben: US 5 277 694, US 5 411 467, US 5 411 467, US 3 764 748, US 4 352 960, US 5 015 225, US 5 015 224, US 3 557 775, US 3 712 962, US 4 988 333, EP 0 263 254, US 5 814 095.

Viele Patienten mit einem Innenohrschaden leiden zusätzlich unter zeitweise auftretenden oder permanenten Ohrgeräuschen (Tinnitus), die operativ nicht behebbar sind und gegen die bis heute keine zugelassenen medikamentösen Behandlungsformen existieren. Daher sind sog. Tinnitus-Maskierer erhältlich; dies sind kleine, batteriebetriebene Geräte, die ähnlich einem Hörgerät hinter oder im Ohr getragen werden und durch artifizielle Schalle, die über einen z.B. Hörgeräte-Lautsprecher in den Gehörgang abgestrahlt werden, den Tinnitus auf psychoakustisch wirkende Weise verdecken ("maskieren") und das störende Ohrgeräusch so möglichst unter die Wahrnehmungsschwelle absenken. Die artifiziellen Schalle sind häufig Schmalbandgeräusche (z.B. Terzrauschen), die in ihrer spektralen Lage und Lautstärkepegel über ein Programmiergerät einstellbar sind, um eine möglichst optimale Anpassung an die individuelle Ohrgeräuschsituation zu ermöglichen. Darüberhinaus existiert seit kurzem die sog. "Retraining-Methode", wobei durch die Kombination eines mentalen Trainingsprogrammes und die Darbietung eines breitbandigen Schalles (Rauschen) nahe der Ruhehörschwelle die Wahrnehmbarkeit des Tinnitus ebenfalls weitgehend unterdrückt werden soll. Diese Geräte werden auch als "Noiser" bezeichnet (Zeitschrift "Hörakustik" 2/97, S. 26, 27).

Bei beiden o.g. Methoden zur apparativen Therapie des Tinnitus sind hörgeräteähnliche, technische Geräte außen am Körper im Ohrbereich sichtbar mitzuführen, die den Träger stigmatisieren und daher nicht gerne getragen werden.

In der US 5 795 287 wird ein implantierbarer Tinnitusmaskierer mit "Direktantrieb" ("direct drive") des Mittelohres z.B. über einen an die Ossikelkette angekoppelten elektromechanischen Wandler beschrieben. Dieser direkt gekoppelte Wandler kann vorzugsweise ein sog. "Floating Mass Transducer" (FMT) sein. Dieser FMT entspricht dem Wandler für implantierbare Hörgeräte, der in der US 5 624 376 beschrieben ist.

In der DE-A-198 58 398 und der DE-A-198 59 171 werden implantierbare Systeme zur Behandlung eines Tinnitus durch Maskierung und/oder Noiserfunktionen beschrieben, bei denen der signalverarbeitende elektronische Pfad eines teil- oder vollimplantierbaren Hörsystems durch entsprechende elektronische Module so ergänzt wird, daß die zur Tinnitusmaskierung oder zur Noiserfunktion notwendigen Signale in den Signalverarbeitungsweg der Hörgerätefunktion einspeisbar sind und die zugehörigen Signalparameter durch weitere elektronische Maßnahmen individuell an die pathologischen Bedürfnisse anpassbar sind. Diese Anpassbarkeit kann dadurch realisiert werden, daß die notwendigen Einstelldaten der Signalerzeugungs- und Einspeiselektronik in demselben physikalischen und logischen Datenspeicherbereich des Implantatsystems hard- und softwaremäßig abgelegt beziehungsweise programmiert werden und über entsprechende elektronische Stellglieder die Einspeisung des Maskierer- beziehungsweise Noisersignals in den Audiopfad des Hörimplantats steuern.

Weitere Systeme zur Tinnitusmaskierung sind beispielsweise aus DE 296 16 956 U1, WO 91/17638, WO96/00051, WO 90/07251, EP 0 537 385 A1, EP 0 400 900 A1, US 5 697 975, US 5 279 292, US 5 788 656 und US 5 403 262 bekannt.

Bei allen o.g. Rehabilitationsgeräten erscheint es heute als sehr sinnvoll, die Systeme so auszulegen, daß sie vollständig implantiert werden können. Solche Hörsysteme bestehen je nach angestrebter Funktion aus drei oder aus vier Funktionseinheiten: ein Sensor (Mikrofon), der den einfallenden Luftschall in ein elektrisches Signal umwandelt, eine elektronische Signalverarbeitungs-, Verstärkungs- und Implantatsteuerungseinheit, ein elektromechanischer beziehungsweise implantierbarer elektroakustischer Wandler, der die verstärkten und vorverarbeiteten Sensorsignale in mechanische beziehungsweise akustische Schwingungen umwandelt und diese über geeignete Koppelmechanismen dem geschädigten Mittel- und/oder Innenohr zuführt oder eine cochleäre Reizelektrode bei Cochleaimplantaten sowie ein elektrisches Energieversorgungssystem, das die genannten Module versorgt. Weiterhin kann eine externe Einheit vorgesehen sein, die dem Implantat elektrische Nachladeenergie zur Verfügung stellt, wenn die implantatseitige Energieversorgungseinheit eine nachladbare (Sekundär-)Batterie enthält. Zweckmäßig ist auch eine Telemetrieeinheit vorzusehen, mit der patientenspezifische, audiologische Daten drahtlos bidirektional übertragen beziehungsweise im Implantat programmiert und damit dauerhaft gespeichert werden können (s. z.B. Zeitschrift HNO 46:853-863 (1998), Leysieffer et al. "Ein vollständig implantierbares Hörsystem für Innenohrschwerhörige: TICA LZ 3001").

Neben den genannten Anwendungsgebieten der vorliegenden Erfindung können als aktive Implantate auch andere Systeme zur Rehabilitation einer beinträchtigen Körperfunktion vorgesehen sein, wie z.B. Herzschrittmacher, Defibrillatoren, Drogenspender, Nerven- oder Knochenwachstumsstimulatoren, Neurostimulatoren, Schmerunterdrückungsgeräte oder dergleichen, bei denen eine sekundäre, wiederaufladbare elektrochemische Zelle als Betriebsenergiequelle verwendet wird.

Bei dem aus DE 41 04 359 C2 bekannten implantierbaren Hörsystem sind gemäß einer ersten Ausführungsform in einem implantierbaren Gehäuse die Steuerelektronik für den Aktor des Hörsystems sowie ein Energiespeicher vorgesehen, der über eine Empfangsspule mittels einer externen Sendespule wiederaufladbar ist. Gemäß einer zweiten Ausführungsform ist die Steuerelektronik in einem separaten implantierbaren Gehäuse untergebracht, welches über eine Kabelsteckverbindung mit dem die Empfangsspule und den wiederaufladbaren Energiespeicher enthaltenden implantierbaren Gehäuse verbunden ist.

Aus DE 198 37 863 C1 ist ein implantierbares Hörsystem bekannt, bei welchem ein elektrochemischer Energiespeicher in einem vorzugsweise hermetisch dichten Gehäuse angeordnet ist, welches wiederum zusammen mit der Steuereinheit und einer TelemetrieEinheit in einem implantierbaren Gehäuse untergebracht ist. Bei dem Energiespeicher kann es sich um eine Primärzelle oder um eine Sekundärzelle handeln, wobei es sich in beiden Fällen um auf Lithium basierende Zellen mit einem festen Polymerelektrolyten handeln kann.

Aus EP 0 981 173 A1 ist ein implantierbares Hörsystem bekannt, bei welchem ein wiederaufladbarer elektrochemischer Energiespeicher in einem hermetisch dichten Gehäuse vorgesehen ist. Eine Überwachungselektronik für das Laden des Energiespeichers sowie eine Empfangsspule für das Laden des Energiespeichers sind in einem separaten Gehäuse untergebracht. Das hermetisch dichte Gehäuse des Energiespeichers ist mit einer mechanischen Überwachungseinheit versehen, die auf Verformung durch Gasaustritt aus dem Speicher mechanisch anspricht und dann den Ladevorgang unterbricht, um eine Beschädigung des Speichers und des Gehäuses durch unzulässige Betriebszustände des Speichers zu verhindern.

Aus DE 198 37 912 C1 ist ein implantierbares Hörsystem bekannt, bei welchem ein mit einem Gehäuse versehener wiederaufladbarer elektrochemischer Energiespeicher in einem hermetisch dichten Gehäuse angeordnet ist, welches mit einer mechanischen Überwachungseinrichtung ausgestattet ist, die auf unzulässigen Gasaustritt aus dem Speicher anspricht und dann gegebenenfalls den Ladevorgang unterbricht um eine Beschädigung des Speichers beziehungsweise des Gehäuses zu verhindern. Das hermetisch dichte Gehäuse ist in einem weiteren hermetisch dichten Gehäuse untergebracht, welches gemäß einer ersten Ausführungsform zusätzlich eine Elektronikeinheit zum Steuern des Lade- beziehungsweise Entladevorgangs, eine Ladestromeinspeise-Anordnung sowie eine zusätzliche elektronische Überwachung der mechanischen Gehäuseüberwachung enthält. Bei einer zweiten Ausführungsform sind diese Komponenten in einem separaten Gehäuse untergebracht, welches auch die Steuerelektronik des Hörsystems enthält. Das hermetisch dichte Gehäuse, welches das hermetisch dichte Gehäuse des Energiespeichers aufnimmt, ist mittels einer lösbaren, starren mechanischen Verbindung mit dem die Steuerelektronik enthaltenden Hauptgehäuse verbunden.

Aus US 6 154 677 ist ein implantierbares Hörsystem bekannt, bei welchem gemäß einer ersten Ausführungsform ein mit einem Gehäuse versehener wiederaufladbarer elektrochemischer Energiespeicher in einem hermetisch dichten Gehäuse untergebracht ist, welches mit einer mechanischen Überwachung versehen ist, die auf unzulässigen Gasaustritt aus dem Speicher anspricht. Gemäß einer ersten Ausführungsform ist dieses hermetisch dichte Speichergehäuse mittels einer Kabelverbindung mit einem implantierbaren Hauptgehäuse verbunden, welches eine Energieempfangsspule, eine entsprechende Elektronik zum Steuern des Lade- beziehungsweise Entladevorgangs sowie die Steuerelektronik für das Hörsystem enthält. Gemäß einer zweiten Ausführungsform ist das hermetisch dichte Speichergehäuse zusammen mit den genannten Komponenten in dem Hauptgehäuse untergebracht.

Aus US 6 227 204 ist ein implantierbares Hörsystem bekannt, bei welchem die Elektronikeinheit zum Überwachen und Steuern des Ladevorgangs so ausgebildet ist, dass das Aufladen des elektrochemischen Energiespeichers in Abhängigkeit von dem Innenwiderstand des Speichers erfolgt, wobei in einer ersten Ladephase ein konstanter Ladestrom fließt und in einer zweiten Ladephase der Ladestrom so eingestellt wird, dass die während des Ladens gemessene Zellenspannung näherungsweise auf einem vorbestimmten konstanten Wert gehalten wird.

Aus DE-A-199 36 063 ist ein implantierbares Hörsystem mit einem wiederaufladbaren elektrochemischen Energiespeicher bekannt, wobei die Elektroden des Speichers direkt, das heißt ohne zusätzliches Gehäuse, in einem hermetisch dichten Gehäuse untergebracht sind, welches von einer mechanischen Einheit überwacht wird, die auf unzulässige Gasentwicklung in dem Gehäuse anspricht und den Ladevorgang dann mechanisch unterbricht. Ferner ist in dem Gehäuse ein Temperatursensor vorgesehen, um den Betriebszustand des Speichers zu überwachen und gegebenenfalls den Ladevorgang mittels einer Überwachungselektronik elektronisch zu unterbrechen. Die Überwachungselektronik kann auch von der mechanischen Überwachungseinheit dazu veranlasst werden, den Ladevorgang zu unterbrechen. Das überwachte, hermetisch dichte Speichergehäuse enthält neben dem Energiespeicher und dem Temperatursensor keine weiteren Komponenten.

Aus der nicht vorveröffentlichten EP-Patentanmeldung mit dem Aktenzeichen 00 119 190.7 sind eine Vorrichtung und ein Verfahren zum Betreiben eines wiederaufladbaren elektrischen Energiespeichers bekannt, wobei die Ladestrategie für den Speicher in Abhängigkeit von einem adaptiven Modell bestimmt wird, welches den Zustand des Speichers vor Inbetriebnahme beschreibende Daten sowie im Betrieb erfassten Daten berücksichtigt und selbsttätig fortlaufend anhand der im Betrieb erfassten Daten optimierbar ist.

Aus der nicht vorveröffentlichten EP-Patentanmeldung mit dem Aktenzeichen 00 119 193.1 ist eine implantierbare Energiespeicheranordnung für ein medizinisches Implantat bekannt, wobei eine von einer Einheit zum Steuern des Ladevorgangs unabhängige Überwachungseinheit vorgesehen ist, welche die Speicherspannung unabhängig von der Steuereinheit erfasst und so ausgebildet ist, dass sie, wenn die von ihr erfasste Speicherspannung außerhalb eines vorbestimmten Bereichs liegt, die Steuerung des Ladepfads übernimmt.

Aus der nicht vorveröffentlichten EP-Patentanmeldung mit dem Aktenzeichen 00 119 194.9 ist eine implantierbare Energiespeicheranordnung für ein medizinisches Implantat bekannt, bei welcher eine Einrichtung vorgesehen ist, welche extern betätigbar ist, um ein Stellglied im Ladepfad zu überbrücken.

Es ist Aufgabe der vorliegenden Erfindung, ein implantierbares, hermetisch dichtes Gehäuse zur Aufnahme eines Energiespeichers sowie einer Elektronikeinheit zu schaffen, welches einfach herzustellen ist, kompakt aufgebaut ist und dennoch für einen hinreichenden Schutz der Elektronikeinheit und des Implantatträgers sorgt. Es ist ferner Aufgabe der vorliegenden Erfindung, ein Herstellungsverfahren für ein solches Gehäuse zu schaffen.

Diese Aufgaben werden erfindungsgemäß gelöst durch ein implantierbares, hermetisch dichtes Gehäuse für Komponenten einer implantierbaren medizinischen Vorrichtung, wobei das Gehäuse eine hermetisch dichte Trennwand aufweist, welche das Gehäuse in eine erste Kammer, die einen Speicher für elektrische Energie für die Stromversorgung der medizinischen Vorrichtung aufnimmt, und eine zweite Kammer unterteilt, die eine Elektronikeinheit aufnimmt, sowie durch Herstellungsverfahren gemäß Anspruch 23 bis 25.

Bei dem erfindungsgemäßen Gehäuse ist vorteilhaft, dass der Energiespeicher und die Elektronikeinheit in einem einzigen Gehäuse aufgenommen sind und dennoch die Elektronikeinheit vor schädlichen Auswirkungen des Energiespeichers, beispielsweise durch Gasaustritt, geschützt ist. Dadurch, dass der Energiespeicher in einer hermetisch dichten Kammer untergebracht ist, ist auch der Implantatträger vor solchen Ereignissen geschützt. Die erfindungsgemäßen Herstellungsverfahren zeichnen sich durch besondere Einfachheit und Zweckmäßigkeit aus.

Bevorzugte Ausgestaltungen der Erfindung ergeben sich aus den Unteransprüchen.

Im folgenden wird die Erfindung anhand der beigefügten Zeichnungen beispielhaft näher erläutert, wobei:
- FIG. 1: schematisch, teilweise im Schnitt dargestellt, eine erste Ausführungsform der Erfindung zeigt; und
- FIG. 2: eine Ansicht wie Fig. 1 zeigt, wobei jedoch eine zweite Ausführungsform der Erfindung dargestellt ist.

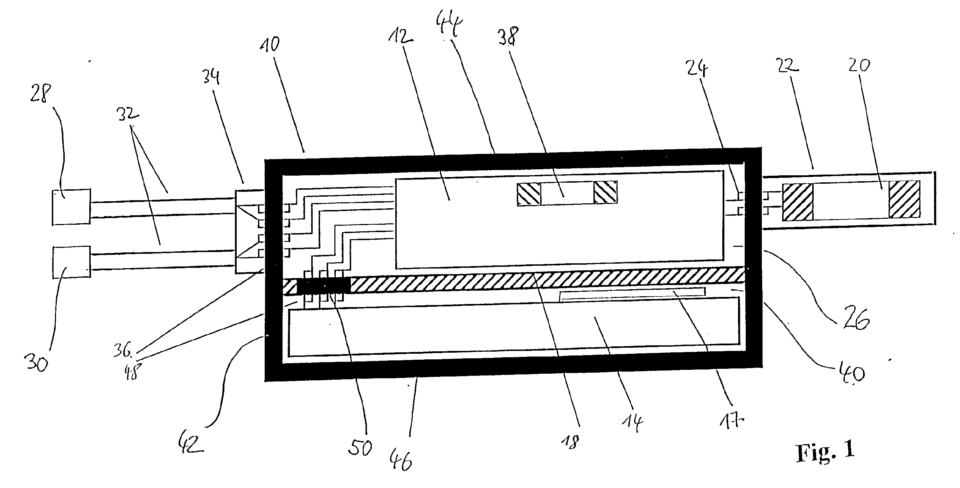

In FIG. 1 ist ein hermetisch dichtes, implantierbares Gehäuse 10 dargestellt, welches vorzugsweise aus Metall gefertigt ist und biokompatibel ist. Als biometallische metallische Werkstoffe kommen Titan, Titanlegierungen, Niob, Nioblegierungen, Tantal oder implantierbare Stähle in Betracht. Alternativ kann jedoch auch eine biokompatible Beschichtung auf der Außenseite vorgesehen sein. Das Gehäuse 10 ist mittels einer hermetisch dichten Trennwand 18 in eine obere Kammer 26 und eine untere Kammer 40 unterteilt, die beide jeweils hermetisch dicht ausgebildet sind. Das Gehäuse 10 kann im wesentlichen zylindrisch ausgebildet sein oder es kann in der Schnittebene von Fig. 1 langgestreckt ausgebildet sein. Die Höhe des Gehäuses 10 ist vorzugsweise geringer als der Durchmesser beziehungsweise die Längsabmessung. Die Trennwand 18 verläuft senkrecht zu der Hochrichtung, das heißt im wesentlichen parallel zu der Gehäuseoberund -unterseite. Vorzugsweise sind die Trennwand 18 und die Seitenwand beziehungsweise Seitenwände 42 aus einem Stück gefertigt, wobei die Kammern 26 und 40 dann jeweils durch einen an der Seitenwand 42 befestigten Deckel 44 beziehungsweise 46 hermetisch dicht verschlossen sind.

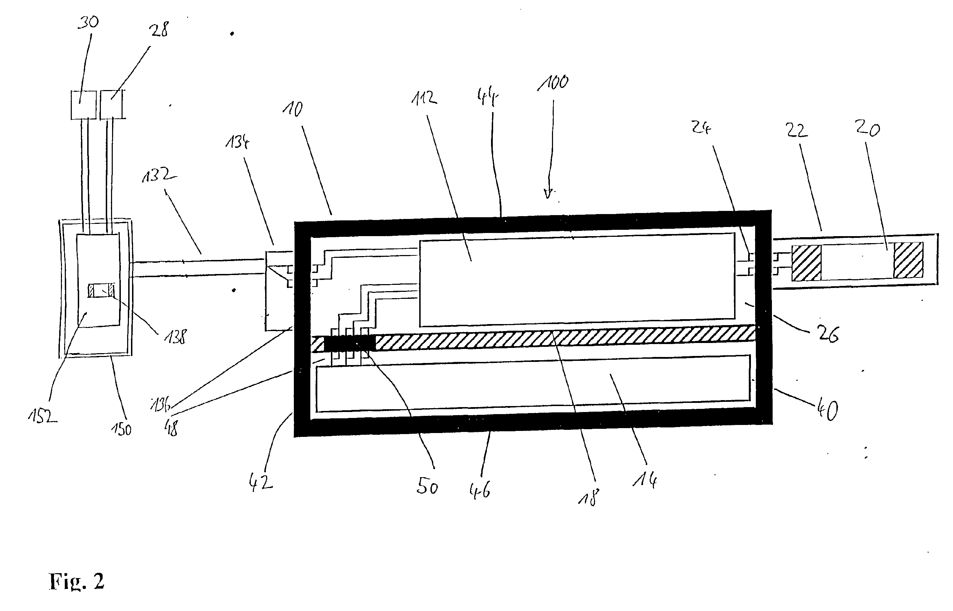

Die obere Kammer 26 nimmt eine Elektronikeinheit 12 nebst einer Telemetriespule 38 auf, während die untere Kammer 40 eine elektrochemische Batterie 14 aufnimmt. Bei der Batterie 14 kann es sich, je nach Strombedarf der Anwendung, um eine Primärbatterie oder um eine wiederaufladbare Sekundärbatterie handeln. In Fig. 1 und 2 ist der Fall gezeigt, wenn es sich um eine Sekundärbatterie handelt. Die Batterie 14 ist in der aus der DE 198 37 863 C1 bekannten Weise mit drei Anschlüssen versehen, nämlich der Kathode, der Anode und einer davon unabhängigen Potentialsonde, wodurch man ein unabhängiges Bezugspotential erhält, mittels dessen sich durch gezielte Überwachung und/oder Regelung einzelner Elektrodenpotentiale relativ zu dem Bezugspotential ungewollte Nebenreaktionen oder ungewollt übermäßig ablaufende Nebenreaktionen an den betrachteten Elektroden erkennen und verhindern lassen. Diese drei Anschlüsse 48 sind mittels einer hermetisch dichten Durchführung 50 durch die Trennwand 18 geführt und mit der Elektronikeinheit 12 kontaktiert.

Die Batterie 14 ist vorzugsweise ohne eigenes Gehäuse direkt in der unteren Kammer 40 aufgenommen, was die Herstellung vereinfacht.

Das Gehäuse 10 kann so hergestellt werden, dass aus einem Rohling aus Vollmaterial mittels spannender Bearbeitung von der Ober- und der Unterseite aus jeweils eine Vertiefung eingesenkt wird, wobei das zwischen den Vertiefungen stehenbleibende Material dann die hermetisch dichte Trennwand 18 bildet. Die so gebildeten Kammern 26 und 40 werden kann durch Aufsetzen und Abdichten der Deckel 44 beziehungsweise 46 hermetisch dicht verschlossen.

Alternativ kann das Gehäuse 10 aus einem flachen Rohling ausgebildet werden, indem aus dem flachen Rohling in einem ersten Tiefziehschritt ein erster offener Hohlraum gebildet wird und in einem zweiten Tiefziehschritt von der gegenüberliegenden Seite aus dem Boden des ersten Hohlraums ein zweiter Hohlraum gebildet wird, wobei die beiden Kammern dann durch Aufsetzen und Abdichten jeweils eines hermetisch dichten Deckels hermetisch abgedichtet werden. Die hermetisch dichte Trennwand wird in diesem Fall von dem Boden des zweiten Tiefziehschritts gebildet.

In einer weiteren alternativen Ausgestaltung kann das Gehäuse 10 aus einem flachen Rohling ausgebildet werden, indem aus dem flachen Rohling in einem Tiefziehschritt ein erster offener Hohlraum mit Boden, das heißt ein Becher, gebildet wird und anschließend auf den Boden des Bechers von der Außenseite ein vorzugsweise zylindrischer Rohrabschnitt aufgesetzt und hermetisch dicht mit dem Boden verbunden wird, um einen zweiten offenen Hohlraum zu bilden. Die beiden hermetisch dichten Kammern werden dann durch Aufsetzen und Abdichten jeweils eines hermetisch dichten Deckels gebildet. Die hermetisch dichte Trennwand wird in diesem Fall von dem Boden des im Tiefziehschritt gebildeten Bechers gebildet. Statt eines an beiden Seiten offenen Rohrabschnitts kann auch ein nur eine offene Seite aufweisender, vorzugsweise becherartiger Hohlkörper (der z.B. mittels eines Tiefziehschritts gebildet sein kann) mit der offenen Seite auf den Boden aufgesetzt werden, wobei in diesem Fall kein Deckel mehr aufgesetzt werden muss, sondern die hermetisch dichte zweite Kammer wird bereits durch das Aufsetzen des Hohlkörpers gebildet.

Es versteht sich, dass in allen Fällen die in den Kammern aufzunehmenden Komponenten vor dem Schritt, welcher die hermetische Abdichtung der jeweiligen Kammer bewirkt, in die noch offene Kammer eingebracht werden müssen.

Bei der Sekundärzelle 14 handelt es sich vorzugsweise um eine auf Lithium basierende Batterie mit festem Elektrolytsystem, beispielsweise einem Polymer-Elektrolytsystem. Bei der Anode der Batterie 14 kann es sich um eine Lithium-Metall- oder Lithium-Legierungs-Elektrode handeln, während es sich bei der Kathode beispielsweise um eine anorganische oder organische Einlagerungs- oder Redox-Elektrode handeln kann. Alternativ kann es sich bei der Anode auch um eine Lithium-Intercallations-Elektrode handeln. Diese Systeme zeichnen sich dadurch aus, dass zumindest bei Vorsehen einer elektronischen Überwachung des Batteriezustands, das heißt einer Überwachung des Batteriezustands durch Überwachung bestimmter elektrischer Parameter, eine schädliche Gasentwicklung verhindert werden kann, welche die Elektronikeinheit 12 gefährden könnte oder zu einem unzulässig hohen Druck im Inneren der Kammer 40 führen könnte.

Zusätzlich zu der nachfolgend geschilderten elektronischen Überwachung der Batterie 14 kann in der unteren Kammer 40 eine Gasbindungseinrichtung 17 vorgesehen sein, die eventuell aus der Batterie 14 austretendes Gas binden, das heißt adsorbieren kann, wobei es sich vorzugsweise um ein Molekularsieb-Adsorptionsmittel handelt (solche Materialien sind auch als Zeolithe bekannt). Auf diese Weise kann aus der Batterie 14 eventuell austretendes Gas zumindest in gewissem Umfang gebunden werden und dadurch der Innendruck der Kammer 40 niedrig gehalten werden.

An einer Schmalseite des hermetisch dichten Gehäuses 10 ist eine Empfangsspule 20 in einer biokompatiblen Polymerhülle 22 angeordnet, wobei die Empfangsspule 20 mittels hermetischen Signaldurchführungen 24 mit der Elektronikeinheit 12 verbunden ist. Die Spule 20 ist so angeordnet, dass sie von der Schmalseite des Gehäuses 10 absteht und in mechanischer Verbindung mit dem Gehäuse 10 steht, beispielsweise durch Verklebung, Anformung oder Anspritzung. Die gezeigte Ausgestaltung der Empfangsspule 20 ist beispielsweise aus US 6 154 677 bekannt. Das Gehäuse 10 kann durch die gezeigte Auslagerung der Spule 20 aus Metall gefertigt sein, wobei die Außenseite biokompatibel beschichtet ist. Die Ladespule 20 dient zum Nachladen der Batterie 14, falls deren Ladezustand eine untere Grenze unterschritten hat, wobei über eine entsprechende Sendespule eines (nicht gezeigten) extrakorporalen Ladegeräts die Empfangsspule 20 transkutan mit elektrischer Energie versorgt wird. Eine solche Anordnung ist beispielsweise aus der US 5 279 292 bekannt.

Die Elektronikeinheit 12 ist so ausgebildet, dass sie eine Einheit umfasst, welche das Laden und Entladen der Batterie 14 überwacht. Dies erfolgt dadurch, dass die Elektronikeinheit 12 beim Ladevorgang den Ladestrom mittels eines Shunt-Widerstands sowie die Spannung der Batterie 14 während des Ladens gemessen werden. Ein darauf aufgebautes Ladeverfahren ist beispielsweise in US 6 227 204 beschrieben, wobei zu Beginn des Ladevorgangs der Ladestrom so gesteuert wird, dass ein relativ hoher, auf einen vorgegebenen Höchstwert begrenzter Ladestrom fließen kann. Sobald die gemessene Batteriespannung (wobei nicht die Leerlaufspannung, sondern die Spannung bei fließendem Ladestrom gemessen wird) einen vorgegebenen Grenzwert erreicht hat, wird der Ladestrom in einer zweiten Ladephase so gesteuert, dass die gemessene Batteriespannung mindestens näherungsweise auf einem vorbestimmten konstanten Wert gehalten wird, welcher mindestens näherungsweise dem Wert der am Ende der ersten Ladephase erreichten Spannung entspricht. Der Ladevorgang wird beendet, sobald die erfasste zeitliche Änderung des Ladestroms einen vorbestimmten Mindestwert unterschreitet. Die Steuerung des Ladestroms kann beispielsweise durch Pulsweitenmodulation oder einen spannungsgeregelten Widerstand erfolgen. Mit diesem Verfahren wird die Aufladung der Batterie abhängig vom Innenwiderstand der Batterie reguliert. Dadurch ist gewährleistet, dass in die Batterie gerade immer nur so viel Energie eingeladen wird, wie es der elektrochemische Zustand erlaubt, ohne dass es dabei zu einer übermäßigen Gasung oder Erwärmung der Zelle kommen kann. Auf diese Weise werden gefährliche Betriebszustände, die zu einer übermäßigen Druckerhöhung im Inneren der Kammer 40 führen können, verhindert werden. Durch die Anpassung der Ladestrategie an den Innenwiderstand der Batterie 14 wird die Ladestrategie automatisch bezüglich Alterungserscheinungen der Zelle angepasst.

Sobald im Betrieb die an der Batterie 14 gemessene Spannung einen vorgegebenen Mindestwert unterschreitet, erzeugt die Elektronikeinheit 12 ein Signal, um den Implantatträger zu veranlassen, einen Ladevorgang vorzunehmen, um eine übermäßige Entladung der Batterie 14 zu verhindern. Konzepte zur Sicherstellung der Wiederaufladbarkeit der Batterie 14 auch im Unterspannungsbereich sind in den nicht vorveröffentlichten EP-Patentanmeldungen mit den Aktenzeichen 00 119 193.1 und 00 119 194.9 beschrieben. Ein Ladekonzept, welches noch flexibler auf zeitliche Veränderungen der Batterieeigenschaften reagieren kann, ist der nicht vorveröffentlichten EP-Patentanmeldung mit dem Aktenzeichen 00 119 190.7 bekannt. Dabei wird im wesentlichen die gesamte Betriebsgeschichte einer speziellen Batterie anhand der Spannungs- und Strommessungen aufgezeichnet und anhand eines adaptiven Modells ausgewertet, wodurch die Ladestrategie ständig aktualisiert und damit optimiert werden kann.

Die bisher beschriebenen Komponenten bilden einen Teil eines implantierbaren Hörsystems, welches eine Sensoreinheit 28, insbesondere in Form eines Mikrophons, sowie eine Aktor-Einheit 30 umfasst, bei welcher es sich beispielsweise um einen an die Gehörknöchelchenkette mechanisch oder an die flüssigkeitsgefüllten Räume des Innenohrs hydromechanisch ankoppelbaren elektromechanischen Wandler handeln kann. Solche Wandler sind beispielsweise in der US 5 277 694, der US 5 411 467 und der US 5 814 095 im einzelnen erläutert und bedürfen daher vorliegend keiner näheren Beschreibung. Die Elektronikeinheit 12 ist dabei so ausgebildet, dass sie die Steuereinheit für den Aktor 30 bildet, wobei es sich im wesentlichen eine Verarbeitungsstufe für die von dem Wandler 28 gelieferten Signale sowie eine Verstärkerstufe handelt, um den Aktor 30 zu betreiben. Die Steuereinheit umfasst ferner einen Microcontroller sowie Analog-Digital-Wandler. Der Microcontroller kann dabei auch für die Überwachung und Steuerung des Ladevorgangs verwendet werden.

Zumindest der Aktor 30 ist als Implantat ausgebildet und ist über Implantatsleitungen 32, einer Steckverbindung 34 sowie hermetischen Signaldurchführungen 36 mit der Elektronikeinheit 12 verbunden. Gleichmaßen ist der Sensor 28, der ebenfalls implantierbar sein kann, über Leitungen 32, die Steckverbindung 34 sowie hermetischen Durchführungen 36 mit der Elektronikeinheit 12 verbunden.

Die Batterie 14 ist vorzugsweise ohne eigenes Gehäuse ausgebildet, sie ist dann vielmehr direkt in der hermetisch dichten Kammer 40 aufgenommen, was die Herstellung des Systems insgesamt vereinfacht. Durch die Wahl eines geeigneten Batterietyps (siehe oben) und durch das Vorsehen einer elektronischen Überwachung des Ladevorgangs sowie gegebenenfalls durch zusätzliche Maßnahmen, wie dem Vorsehen einer Gasbindeeinrichtung, kann das Austreten von unzulässigen Gasmengen aus der Batterie 14 zuverlässig verhindert werden. Insofern ist auch keine redundante mechanischen Überwachung des hermetisch dichten Gehäuses 10, beispielsweise durch einen auf einen Druckanstieg im Gehäuseinneren ansprechenden mechanischen Sensor und Schalter, erforderlich, was einerseits einen kompakten Aufbau des Gehäuses 10 sowie ferner eine vereinfachte Fertigung ermöglicht.

Bei der Batterie 14 kann es sich bei Anwendungen mit geringem Stromverbrauch statt um eine (wiederaufladbare) Sekundär-Batterie auch um eine (nicht wiederaufladbare) Primär-Batterie handeln, wobei dann natürlich keine Überwachungsfunktion für einen Ladevorgang implementiert ist. Statt dessen kann die Elektronikeinheit 12 mit einer Funktion versehen sein, welche den Ladezustand der Primär-Batterie anzeigt, beispielsweise die noch verbleibende Betriebsdauer bis zur Erschöpfung der Batterie.

Die Datentelemetriespule 38 ist vorgesehen, um einen Datenaustausch mit einer extrakorporalen Datensendeeinrichtung zu ermöglichen. Auf diese Weise kann beispielsweise das den Betrieb des Aktors 30 steuernde Programm bei Bedarf aktualisiert beziehungsweise an Gegebenheiten des Implantatträgers angepasst werden. Eine solche Datenbeziehungsweise Programmaktualisierung kann auch das Ladevorgangs-Überwachungsprogramm betreffen.

In Fig. 2 ist eine alternative Ausführungsform gezeigt, welche sich von der Ausführungsform gemäß Fig. 1 im wesentlichen dadurch unterscheidet, dass der den Betrieb des Aktors 30 steuernde Teil der Elektronikeinheit in einem separaten biokompatiblen, implantierbaren, hermetisch dichten Gehäuse 150 untergebracht ist. Diese Steuerelektronik ist mit dem Bezugszeichen 152 bezeichnet. Das Gehäuse 150 nimmt ferner die Datentelemetriespule 138 auf. Die Steuerelektronik 152 wird über Leitungen 132, eine Steckverbindung 134 sowie hermetische Durchführungen 136 mit der Ladeelektronik 112 verbunden, welche die vorstehend geschilderten Überwachungs- und Steuerfunktionen beim Ladevorgang der Batterie 14 übernimmt. Der Temperatursensor 26, die Gasbindungseinrichtung 16 sowie die Lade-Empfangsspule 20 sind wie in Fig. 1 ausgebildet. Bei der Ausführungsform gemäß Fig. 2 bildet das Gehäuse 10 mit den darin aufgenommenen beziehungsweise daran angebrachten Komponenten ein Energieversorgungsmodul 100 für die Steuereinheit 152. Statt über eine steckbare Kabelverbindung 132 kann das Energieversorgungsmodul 100 auch direkt mit dem Gehäuse 150 für die Steuereinheit 152 verbunden sein, wobei in diesem Fall ein Koppelorgan vorgesehen ist, welches für eine lösbare, starre mechanische Anbindung des Energieversorgungsmoduls 100 an das Gehäuse 150 sorgt. Ein solches Koppelorgan dient gleichzeitig einer lösbaren, galvanischen Kontaktierung der Batterie 14.

Für den Fall, dass es sich bei der Batterie 14 um eine Primär-Batterie handelt, kann die Elektronikeinheit 112, wie im Zusammenhang mit der Ausführungsform von Fig. 1 beschrieben, statt mit einer Ladevorgangsüberwachungsfunktion mit einer Ladezustandsanzeigefunktion versehen sein.

Die Steuereinheit 12 beziehungsweise 112 kann so ausgebildet sein, dass sie die Energieabgabe in der Batterie 14 steuert beziehungsweise auf die einzelnen Verbraucher aufteilt.

## Patentansprüche

1. Implantierbares, hermetisch dichtes Gehäuse (10) für Komponenten (12, 112, 14, 38) einer implantierbaren medizinischen Vorrichtung, wobei das Gehäuse eine hermetisch dichte Trennwand (18) aufweist, welche das Gehäuse in eine erste Kammer (40), die einen Speicher (14) für elektrische Energie für die Stromversorgung der medizinischen Vorrichtung aufnimmt, und eine zweite Kammer (26), die eine Elektronikeinheit (12, 112) aufnimmt, unterteilt.

2. Vorrichtung nach Anspruch 1, **dadurch gekennzeichnet, dass** die Trennwand (18) mit hermetisch dichten elektrischen Durchführungen (50) versehen ist.

3. Vorrichtung nach Anspruch 1 oder 2, **dadurch gekennzeichnet, dass** die Trennwand (18) im wesentlichen in Richtung des größten Abmessung des Gehäuses (10) verläuft.

4. Vorrichtung nach Anspruch 3, **dadurch gekennzeichnet, dass** das Gehäuse (10) im wesentlichen zylindrisch oder langgestreckt ausgebildet ist, wobei der Durchmesser bzw. die Längsabmessung größer als die Höhe ist.

5. Vorrichtung nach Anspruch 4, **dadurch gekennzeichnet, dass** die Umfangswand (36) und die Trennwand (18) aus einem Stück gefertigt sind und die erste (40) und die zweite Kammer (26) jeweils durch einen an der Umfangswand befestigten Deckel (44, 46) verschlossen sind.

6. Vorrichtung nach einem der vorhergehenden Ansprüche, **dadurch gekennzeichnet, dass** der Energiespeicher (14) ohne eigenes Gehäuse unmittelbar in der erste Kammer (40) aufgenommen ist.

7. Vorrichtung nach Anspruch 6, **dadurch gekennzeichnet, dass** es sich bei dem Speicher (14) um eine elektrochemische Batterie mit festem Elektrolytsystem, insbesondere um eine Lithium-basierende Batterie, handelt.

8. Vorrichtung nach einem der vorhergehenden Ansprüche, **dadurch gekennzeichnet, dass** es sich bei der Elektronikeinheit (112) um eine Einheit zur Überwachung des Speichers (14) und/oder zum Steuern der medizinischen Vorrichtung handelt.

9. Vorrichtung nach einem der vorhergehenden Ansprüche, **dadurch gekennzeichnet, dass** der Speicher eine Primärbatterie ist.

10. Vorrichtung nach Anspruch 1 bis 8, **dadurch gekennzeichnet, dass** der Speicher (14) eine Sekundärbatterie ist.

11. Vorrichtung nach Anspruch 10, **dadurch gekennzeichnet, dass** die Elektronikeinheit (12, 112) so ausgebildet ist, dass sie den Ladevorgang des Energiespeichers (14) so überwacht und steuert, dass der Betriebszustand des Energiespeichers in einem festgelegten Bereich gehalten wird, in welchem eine Beschädigung des Energiespeichers und ein Austritt von Gas im wesentlichen ausgeschlossen ist.

12. Vorrichtung nach Anspruch 11, **dadurch gekennzeichnet, dass** die Elektronikeinheit (12, 112) so ausgebildet ist, dass sie den Ladevorgang unterbricht, wenn der Betriebszustand des Energiespeichers (14) den festgelegten Bereich zu verlassen droht.

13. Vorrichtung nach Anspruch 10 bis 12, **dadurch gekennzeichnet, dass** eine Empfangsspule (20) vorgesehen ist, in die über eine extrakorporal angeordnete Ladevorrichtung Energie zum Nachladen des Speichers (14) transkutan elektromagnetisch einspeisbar ist, und die vorzugsweise an der Außenseite, insbesondere der Schmalseite, des hermetisch dichten Gehäuses (10) in einer biokompatiblen Polymerhülle (22) in mechanischer Verbindung mit dem Gehäuse angeordnet ist.

14. Vorrichtung nach einem der vorhergehenden Ansprüche, **dadurch gekennzeichnet, dass** die Elektronik-Einheit (12) eine Spule (38) für den Austausch von Daten für die Steuerung der medizinischen Vorrichtung mit einer extrakorporalen Telemetrie-Einheit aufweist.

15. Vorrichtung nach einem der vorhergehenden Ansprüche, **dadurch gekennzeichnet, dass** das hermetisch dichte Gehäuse (10) an der Außenseite mit durchgeführten Anschlüssen für eine sensorische Komponente (28) und eine aktorische Komponente (30) versehen ist.

16. Vorrichtung nach einem der vorhergehenden Ansprüche, **dadurch gekennzeichnet, dass** es sich bei der medizinischen Vorrichtung um eine Hörhilfe handelt.

17. Verfahren zum Herstellen eines hermetisch dichten Gehäuses (10) gemäß einem der vorhergehenden Ansprüche, wobei aus einem Rohling mittels spanender Bearbeitung von zwei gegenüber liegenden Seiten aus zwei kammerartige Vertiefungen ausgebildet werden, wobei das zwischen den Vertiefungen stehenbleibende Material die hermetisch dichte Trennwand (18) bildet und wobei die erste (40) und die zweite Kammer (26) durch Aufsetzen jeweils eines hermetisch dichten Deckels (44, 46) auf die beiden Vertiefungen gebildet wird.

18. Verfahren zum Herstellen eines hermetisch dichten Gehäuses gemäß einem der Ansprüche 1 bis 16, wobei aus einem flachen Rohling in einem ersten Tiefziehschritt ein erster offener Hohlraum gebildet wird, in einem zweiten Tiefziehschritt von der gegenüberliegenden Seite aus dem Boden des ersten Hohlraums ein zweiter offener Hohlraum gebildet wird und die erste und die zweite Kammer durch Aufsetzen jeweils eines hermetisch dichten Deckels auf die Öffnungen der beiden Hohlräume gebildet wird.

19. Verfahren zum Herstellen eines hermetisch dichten Gehäuses gemäß einem der Ansprüche 1 bis 16, wobei aus einem flachen Rohling in einem Tiefziehschritt ein erster offener Hohlraum mit einem Boden gebildet wird, anschließend ein an mindestens einer Seite offener Hohlkörper, insbesondere ein Rohrabschnitt, mit der bzw. einer offenen Seite von außen auf den Boden aufgesetzt und hermetisch dicht mit diesem verbunden wird, wobei die erste Kammer durch Aufsetzen eines hermetisch dichten Deckels auf die Öffnung der ersten Hohlraums gebildet wird und die zweite Kammer entweder durch das Aufsetzen des Hohlraums oder, wenn der Hohlkörper eine zweite offene Seite aufweist, durch Aufsetzen eines hermetisch dichten Deckels auf die noch offene Seite des Hohlkörpers gebildet wird.
